# EUROPÄISCHE PATENTSCHRIFT

(11) **EP 4 488 256 B1**
(45) Veröffentlichungstag und Bekanntmachung des Hinweises auf die Patenterteilung: **08.04.2026**
(21) Anmeldenummer: 23183874.9
(22) Anmeldetag: 06.07.2023
(51) Int. Cl.: C07C 43/04, C07C 41/09, C07C 29/152, C07C 31/04, B01J 19/24, B01J 8/04

(54) **VERFAHREN ZUM HERSTELLEN VON DIMETHYLETHER (DME) AUS SYNTHESEGAS**
PROCESS FOR THE PRODUCTION OF DIMETHYL ETHER (DME) FROM SYNTHESIS GAS
PROCÉDÉ DE PRÉPARATION DE DIMÉTHYLÉTHER (DME) À PARTIR DE GAZ DE SYNTHÈSE

(43) Veröffentlichungstag der Anmeldung: 08.01.2025
(73) Patentinhaber: L'AIR LIQUIDE, SOCIETE ANONYME POUR L'ETUDE ET L'EXPLOITATION DES PROCEDES GEORGES CLAUDE, 75007 Paris (FR)
(72) Erfinder: Haag, Stephane, 60388 Frankfurt am Main (DE); Castillo-Welter, Frank, 60388 Frankfurt am Main (DE); Renner, Thomas, 60388 Frankfurt am Main (DE); Lin, Lin, 60388 Frankfurt am Main (DE)
(74) Vertreter: Air Liquide

(56) Entgegenhaltungen:
- WO-A1-2019/122078
- DE-A1- 102016 005 999
- ZHU Y ET AL: "Experimental study of improved two step synthesis for DME production", FUEL PROCESSING TECHNOLOGY, ELSEVIER BV, NL, vol. 91, no. 4, 1 April 2010 (2010-04-01), pages 424 - 429, XP026963432, ISSN: 0378-3820, [retrieved on 20090524]
- EKSIRI Z ET AL: "Two-dimensional modeling investigation of the modern methanol plate reactors", CHEMICAL ENGINEERING RESEARCH AND DESIGN, ELSEVIER, AMSTERDAM, NL, vol. 162, 19 August 2020 (2020-08-19), pages 212 - 227, XP086259149, ISSN: 0263-8762, [retrieved on 20200819], DOI: 10.1016/J.CHERD.2020.08.009

## Beschreibung

### Gebiet der Erfindung

Die Erfindung betrifft ein Verfahren zum Herstellen von Dimethylether (DME) aus einem Wasserstoff und Kohlenoxide enthaltenden Synthesegas.

### Stand der Technik

Die katalytische Herstellung von Dimethylether (DME) aus Methanol durch katalytische Dehydratisierung ist seit vielen Jahren bekannt. So beschreibt das US-Patent US 2014408 ein Verfahren zur Herstellung von DME aus Methanol an Katalysatoren wie Aluminiumoxid, Titanoxid und Bariumoxid, wobei Temperaturen von 350 bis 400°C bevorzugt sind.

Ein Artikel von Yingying Zhu et al., Fuel Processing Technology, Band 91, Ausgabe 4, 2010, Seiten 424-429, beschreibt eine zweistufige Synthese zur Herstellung von DME, umfassend eine Methanolsynthese und eine Methanoldehydratisierung in einem Festbettreaktor.

Weitere Informationen zum Stand der Technik und zur heutigen Praxis der Herstellung von Dimethylether finden sich in Ullmann's Encyclopedia of Industrial Chemistry, Sixth Edition, 1998 Electronic Release, Stichwort "Dimethyl Ether". Insbesondere wird dort in Kapitel 3 "Production" dargelegt, dass die katalytische Umsetzung von reinem, gasförmigem Methanol in einem Festbettreaktor durchgeführt wird.

Aus reaktionstechnischer Sicht werden für die katalytische Dehydratisierung von Methanol zu DME in der Gasphase Festbettreaktoren bevorzugt eingesetzt, da sie sich durch konstruktive Einfachheit auszeichnen. So beschreibt die deutsche Offenlegungsschrift DE 3817816 ein in eine Methanol-Syntheseanlage integriertes Verfahren zur Herstellung von Dimethylether durch katalytische Dehydratisierung von Methanol ohne vorherige Abtrennung des im Methanolreaktor nicht umgesetzten Synthesegases. Als Dehydratisierungsreaktor wird ein einfacher Festbettreaktor verwendet. Wird dieser ohne zusätzliche Maßnahmen zur Temperaturregelung ausgeführt, sondern lediglich zur Vermeidung von Wärmeverlusten mit einer äußeren Isolierung umgeben, so bezeichnet man ihn auch als adiabaten Festbettreaktor.

Bei der Dehydratisierung von Methanol zu Dimethylether gemäß der Reaktionsgleichung

2 CH₃OH = (CH₃)₂O + H₂O

handelt es sich um eine exotherme Gleichgewichtsreaktion; daraus folgt, dass hohe Umsatzgrade aus thermodynamischer Sicht bei möglichst niedrigen Reaktionstemperaturen erzielt werden. Andererseits wird aus reaktionskinetischer Sicht eine Mindestreaktionstemperatur benötigt, um ausreichende Reaktionsgeschwindigkeiten, und somit akzeptable Methanolumsätze, zu gewährleisten. Nachteilig bei den bislang verwendeten adiabaten Festbettreaktoren ist die fehlende Möglichkeit, eine optimale Temperaturführung zur Gewährleistung hoher Umsatzgrade und zur Minimierung der Bildung von Nebenprodukten zu gewährleisten.

Die Bildung von Nebenprodukten, wie beispielsweise Kohlenmonoxid CO, Kohlendioxid CO₂, Wasserstoff H₂ und Methan CH₄, erfolgt vorzugsweise bei höheren Temperaturen. Als mögliche Ursache für die Bildung der drei erstgenannten Nebenprodukte wird die Dampfspaltung von Methanol im Einsatzstrom oder bereits gebildetem DME mit Wasserdampf vermutet, der als Reaktionsnebenprodukt entsteht. Methan kann beispielsweise durch Folgereaktion der gebildeten Kohlenoxide mit Wasserstoff entstehen. Die Bildung dieser Nebenprodukte ist unerwünscht, da sie die Reinheit des Reaktionsproduktes beeinträchtigen und die Selektivität der Reaktion zu DME reduzieren.

Die theoretische Studie "Modeling and Optimization of MeOH to DME Isothermal Fixed-bed Reactor", Farsi et al., International Journal of Chemical Reactor Engineering, Volume 8, 2010, Article A79, beschreibt den optimalen Temperaturverlauf der Reaktortemperatur für die katalytische Dehydratisierung von Methanol zu Dimethylether in einem quasi- (oder weitgehend) isothermen Festbettreaktor, in dem der feste Katalysator in Röhren angeordnet ist, die auf der Mantelseite von teilweise verdampfendem Wasser als Kühlmedium umströmt werden. Unter Verwendung eines genetischen Algorithmus, der thermodynamische und kinetische Gesichtspunkte der Dehydratisierungsreaktion berücksichtigt, wird ein vom Reaktoreintritt zum Reaktoraustritt exponentiell abfallendes Temperaturprofil als Optimum berechnet, wobei die Reaktoreintrittstemperatur ca. 800 K und die Reaktoraustrittstemperatur ca. 560 K beträgt. Unter Zugrundelegung dieses axialen Temperaturverlaufs wird für den optimierten isothermen Reaktor ein Methanolumsatz von rund 86 % berechnet, während er beim adiabaten Reaktor lediglich ca. 82 % beträgt. Allerdings gibt die genannte Arbeit keine Informationen, wie ein optimierter Festbettreaktor zur DME-Herstellung aus Methanol konstruktiv auszugestalten ist. Auch wird lediglich der Methanolumsatz, nicht aber die Bildung etwaiger Nebenprodukte, als Optimierungskriterium herangezogen.

Nachteilig bei den bislang erörterten Verfahren zur Herstellung von DME ist es, dass in aufwändiger Weise zunächst Methanol hergestellt und isoliert werden muss. In einem zweiten Schritt erfolgt dann die Umsetzung des Methanols zum Zielprodukt DME. Insgesamt ergeben sich bei zweistufigen Verfahren zur DME-Synthese die Nachteile, dass die benötigte Syntheseanlage zwei verschiedene Reaktoren umfasst, so dass mehr Ausrüstungspositionen erforderlich sind und der apparative Aufwand beträchtlich ist. Ferner ist es erforderlich, das im ersten Syntheseschritt erzeugte Methanol abzutrennen sowie zu reinigen und zu kühlen.

Es wurden daher bereits im Stand der Technik Verfahren zur sog. DME-Direktsynthese erörtert, in denen das Zwischenprodukt Methanol nicht auftritt und/oder nicht isoliert wird, sondern die Synthese verläuft, ausgehend von Synthesegas, direkt bis zum Zielprodukt DME. Beispielsweise offenbart die US-Patentschrift US 10501394 B2 ein Verfahren zur Herstellung von DME aus Synthesegas, bei dem mindestens ein Strom aus Synthesegas mindestens einem Syntheseschritt unterzogen wird, bei dem die im Einsatzstrom vorhandenen Komponenten zumindest teilweise in Dimethylether (DME) umgewandelt werden, wobei man mindestens einen Rohproduktstrom erhält, der zumindest DME und die nicht umgesetzten Komponenten des Einsatzstroms enthält. Der mindestens eine Syntheseschritt wird dabei unter isothermen Bedingungen durchgeführt.

Solche Verfahren zur einstufigen Synthese oder DME-Direktsynthese weisen allerdings folgende Nachteile auf:
- Bei der einstufigen Synthese werden viele Nebenprodukte erzeugt, beispielsweise Methanol, Methan, Kohlendioxid, geringe Mengen C2+ Kohlenwasserstoffe sowie nicht umgesetzte Synthesegasbestandteile.
- Es müssen mehr nicht umgewandelte Verbindungen in den Synthesereaktor zurückgeführt werden, um eine hohe DME-Ausbeute sicherzustellen.
- Die Selektivität der einstufigen Synthese für DME ist zumeist schlecht.
- Das H₂/CO-Verhältnis des Synthesegases hat einen großen Einfluss auf die Selektivität.
- Die Lebensdauer des Katalysators ist häufig zu klein.

### Beschreibung der Erfindung

Es ist daher die Aufgabe der vorliegenden Erfindung, ein Verfahren zur Herstellung von DME vorzuschlagen, die die genannten Nachteile einstufiger und zweistufiger Herstellungsverfahren Verfahren vermeidet, die bislang aus dem Stand der Technik bekannt sind. Diese Aufgabe wird in einem ersten Aspekt der Erfindung durch ein Verfahren mit den Merkmalen des Anspruchs 1 gelöst. Weitere Aspekte der Erfindung ergeben sich aus den abhängigen Verfahrensansprüchen.

Die für die Umsetzung von Synthesegas zu Methanol erforderlichen Methanolsynthesebedingungen und die für die Umsetzung von Methanol zu DME erforderlichen DME-Synthesebedingungen sind dem Fachmann aus dem Stand der Technik, beispielsweise den eingangs erörterten Druckschriften, bekannt. Es sind diejenigen physikalisch-chemischen Bedingungen, unter denen ein messbarer, bevorzugt ein technisch relevanter Umsatz von Synthesegas zu Methanol bzw. von Methanol zu DME erzielt wird. Notwendige Anpassungen dieser Bedingungen an die jeweiligen Betriebserfordernisse wird der Fachmann auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegende Anmeldung sind die Aggregatzustände gasförmig bzw. dampfförmig als synonym zu betrachten.

Thermische Trennverfahren im Sinne der vorliegenden Erfindung sind alle Trennverfahren, die auf der Einstellung eines thermodynamischen Phasengleichgewichtes beruhen. Bevorzugt sind dies die Destillation oder Rektifikation mit jeweils mehrfacher Einstellung des Dampf-Flüssig-Gleichgewichtes oder das Auftrennen eines Gas-Flüssig-Gemisches bzw. Dampf- Flüssig-Gemisches in einer Phasentrennvorrichtung mit einfacher Einstellung des Dampf-Flüssig-Gleichgewichtes. Grundsätzlich ist aber auch der Einsatz anderer thermischer Trennverfahren denkbar, beispielsweise der Absorption, Extraktion oder Extraktivdestillation.

Unter einem Aufteilen oder Auftrennen bzw. Abtrennen eines Stoffstroms ist im Zusammenhang mit der vorliegenden Erfindung die Erzeugung mindestens zweier Teilströme aus dem ursprünglichen Stoffstrom zu verstehen, wobei mit dem Auftrennen bzw. Abtrennen eine beabsichtigte Änderung der stofflichen Zusammensetzung der erhaltenen Teilströme in Bezug auf den ursprünglichen Stoffstrom verbunden ist, beispielsweise durch Anwendung eines thermischen Trennverfahrens oder zumindest thermischen Trennschrittes auf den ursprünglichen Stoffstrom. Dagegen ist mit dem Aufteilen des ursprünglichen Stoffstroms in der Regel keine Änderung der stofflichen Zusammensetzung der erhaltenen Teilströme verbunden.

Eine Thermoplatte im Sinne der Erfindung besteht aus zwei Blechen (Thermoblechen), welche an den Rändern zusammengeschweißt sind und über deren Oberfläche eine Vielzahl von Punktschweißungen, die die Platten ebenfalls miteinander verbinden, verteilt sind. Solche Thermoplatten können automatisiert von Robotern und somit zu sehr günstigen Preisen hergestellt werden. Nach der Verschweißung werden die Bleche durch eine hydraulische Umformung, in der Regel also das Einpressen einer Flüssigkeit unter hohem Druck, expandiert, wodurch kissenartige Kanäle zwischen den Blechen entstehen, die von einem Kühlfluid oder Heizfluid frei durchströmbar sind. Die Thermoplatten können dabei parallel nebeneinander auf der horizontalen Achse oder orthogonal zur horizontalen Achse in dem Synthesereaktor orientiert sein. Der Freiraum zwischen zwei Thermoplatten kann mit einer Schüttung eines festen, körnigen Katalysators gefüllt werden. Bei entsprechender Anordnung von drei oder mehr Thermoplatten ergibt sich dabei eine sandwichartige Struktur mit guter Zugänglichkeit des Katalysators für ein- und ausströmende Stoffströme, beispielsweise ein Einsatzgas und ein Produktgas, sowie eine sehr gute Wärmeübertragung zwischen den Katalysatorschüttungen und dem einem Kühlfluid oder Heizfluid.

Gegenstand der Erfindung ist es, die zweistufige DME-Synthese ohne Isolierung des Zwischenproduktes Methanol in einem gemeinsamen Synthesereaktor durchzuführen, um möglichst die Vorteile der beiden Synthesewege zu erhalten bzw. deren Nachteile zu minimieren. Dazu werden eine erste Reaktionszone, in der Synthesegas zu Methanol umgewandelt wird, mit einer zweiten Reaktionszone kombiniert, in der Methanol zu DME weiterreagiert. Beide Reaktionszonen werden in einem gemeinsamen, drucktragenden Reaktormantel (Mantelrohr) angeordnet und es erfolgt keine Isolierung und Reinigung des intermediär erzeugten Methanols.

Beide Reaktionsschritte erfolgen an Schüttungen eines für die jeweilige Teilreaktion spezifischen, festen, partikelförmigen Katalysators. Gegenstand der Erfindung ist es, dass die Katalysatorschüttungen jeweils zwischen zwei benachbarten Thermoplatten angeordnet sind und von dem jeweiligen Einsatzgas durchströmbar sind. Die Thermoplatten sind dabei für ein fluides Kühlmedium durchströmbar. Wegen der sandwichartigen Anordnung von Katalysatorschichten und Kühlschichten wird eine sehr gute Temperaturlenkung innerhalb des Katalysatorbetts erreicht, so dass isotherme Verhältnisse in den einzelnen Katalysatorbetten erreicht oder zumindest angenähert werden. Insbesondere werden sog. Hot Spots in den Katalysatorbetten, also Zonen lokaler hoher Temperatur, vermieden oder reduziert. Hot Spots wirken sich nachteilig auf die mögliche Betriebsdauer der Katalysatoren aus und verringern bei exothermen Gleichgewichtsreaktionen den integral über ein Katalysatorbett erzielbaren Umsatz der Eduktkomponenten.

Der den Synthesereaktor verlassende, DME enthaltende Produktgasstrom wird zu einer nach mindestens einem thermischen Trennverfahren arbeitenden Trennvorrichtung geführt; dies kann beispielsweise eine einstufige oder mehrstufige Destillation sein. In der Trennvorrichtung erfolgt ein Auftrennen des DME enthaltenden Produktgasstroms in einen DME-Endproduktstrom, einen Gas-Nebenproduktstrom, der nicht umgesetzte Kohlenoxide und Wasserstoff enthält, einen Methanol-Nebenproduktstrom und einen Abwasserstrom. Der Gas-Nebenproduktstrom wird dabei mindestens teilweise zum Reaktoreingang zurückgeführt, um die insgesamt erzielte DME-Ausbeute zu steigern.

Da eine Kontrolle der Reaktionstemperaturen und des Drucks von Bedeutung ist, um eine maximale Umwandlung zu erreichen, beschäftigen sich verschiedene Ausgestaltungen der Erfindung mit der Nutzung unterschiedlicher Stoffströme als Kühlmittel und mit deren Führung durch den Synthesereaktor. Als Kühlmittel können spezielle Wärmeträgerfluide, aber auch die Edukt- und/oder Produktströme der Reaktionszonen verwendet werden.

Der Druck im erfindungsgemäßen Synthesereaktor beträgt dabei 40 bis 90 bar, absolut, bevorzugt 60 bis 80 bar, absolut. Bevorzugt wird ein Synthesegas, dessen CO-Konzentration höher ist als dessen CO₂-Konzentration, da hiermit weniger Wasser entsteht und die Selektivität zu DME besser ist. Es können aber auch andere Synthesegase verwendet werden, beispielsweise auch Gemische von CO₂ und Wasserstoff mit nur geringer oder keiner Beimischung von CO.

Bevorzugt beträgt die Temperatur in der ersten Reaktionszone zwischen 180 und 350 °C, meist bevorzugt zwischen 200 und 280 °C.

Bevorzugt beträgt die Temperatur in der zweiten Reaktionszone zwischen 220 und 350 °C, weiter bevorzugt zwischen 240 und 320 °C, meist bevorzugt zwischen 260 und 300 °C. Untersuchungen haben ergeben, dass in diesen Temperaturbereichen hohe Ausbeuten an DME und geringe Ausbeuten an Nebenprodukten erhalten werden.

Als Kühlmittel können spezielle Wärmeträgerfluide, aber auch die Edukt- und/oder Produktströme der Reaktionszonen verwendet werden. In einem ersten Beispiel wird Wasser als Kühlmedium verwendet, wobei beispielsweise Frischwasser oder bei der Reaktion erzeugtes Wasser oder Mischungen beider verwendet werden können. In einem zweiten Beispiel wird kaltes Synthesegas als Kühlmedium verwendet, das dabei selber vorgewärmt wird, so dass sich der Bedarf des Verfahrens an Heizenergie verringert. In einem dritten Beispiel wird ein Methanol-Nebenproduktstrom aus der Trennvorrichtung als Kühlmedium verwendet. Hierdurch verringert sich der Energiebedarf der nachgeschalteten Herstellung von Reinmethanol.

Vorteilhaft bei dem erfindungsgemäßen Verfahren ist seine schnelle Anfahrmöglichkeit: Hierzu wird zunächst die zweite Reaktionszone (DME-Synthese) mit von außen zugeführtem Methanol in Betrieb genommen und Synthesegas als Kühlmedium durch die zweite Reaktionszone geführt, um die gewünschte Temperatur zu erreichen und sodann die die erste Reaktionszone (Methanolsynthese) in Betrieb nehmen zu können.

Vorteilhaft bei dem erfindungsgemäßen Verfahren ist es, dass mit dem Synthesereaktor eine Minimierung von Nebenprodukten bei gleichzeitiger Erzielung eines gleichgewichtsnahen Umsatzes (40 % DME, 40 % H₂O, 20 % nicht umgewandeltes Methanol) ermöglicht wird. Die als Zwischenprodukte (Methanol) bzw. Endprodukte (DME, CO, CO₂, Wasserstoff und Methan) sind keine Katalysatorgifte für die verwendeten, an sich handelsüblichen Katalysatoren.

Eine maximale Reaktionstemperatur von 400 °C sollte in der zweiten Reaktionszone nicht überschritten werden, um das Ausmaß der Methanisierung gering zu halten.

### Weitere Aspekte der Erfindung

Ein zweiter Aspekt des erfindungsgemäßen Verfahrens ist dadurch gekennzeichnet, dass ein erster Teil des Gas-Nebenproduktstroms zu dem DME-Synthesereaktor zurückgeführt und gemeinsam mit dem ersten Einsatzgasstrom in den DME-Synthesereaktor eingeleitet wird. Hierdurch wird der Umsatz an Eduktkomponenten und die DME-Ausbeute erhöht.

Ein dritter Aspekt des erfindungsgemäßen Verfahrens ist dadurch gekennzeichnet, dass ein zweiter Teil des Gas-Nebenproduktstroms als Spülstrom (Purge) aus dem Verfahren ausgeleitet wird. Auf diese Weise wird eine Akkumulation etwaiger Inertkomponenten im Sinne der beiden Teilreaktionen, beispielsweise von Methan, verhindert.

Ein vierter Aspekt des erfindungsgemäßen Verfahrens ist dadurch gekennzeichnet, dass mindestens ein Teil des Methanol-Nebenproduktstroms zu dem DME-Synthesereaktor zurückgeführt und in den Zwischenraum und/oder in die Katalysatorschüttungen in der zweiten Reaktionszone eingeleitet wird. Auf diese Weise kann speziell die Ausbeute bei der zweiten Teilreaktion, der DME-Synthese, erhöht werden.

Ein fünfter Aspekt des erfindungsgemäßen Verfahrens ist dadurch gekennzeichnet, dass als erstes fluides Kühlmedium ein erster Kühlwasserstrom und als zweites fluides Kühlmedium ein zweiter Kühlwasserstrom verwendet wird. Hierdurch können die optimalen Temperaturen in der ersten und zweiten Reaktionszone leicht eingestellt werden.

Ein sechster Aspekt des erfindungsgemäßen Verfahrens ist dadurch gekennzeichnet, dass als erstes fluides Kühlmedium und als zweites fluides Kühlmedium ein gemeinsamer Kühlwasserstrom verwendet wird. Dieser Aspekt gestattet einen ressourcenschonenden Einsatz von Kühlwasser.

Ein siebter Aspekt des erfindungsgemäßen Verfahrens ist dadurch gekennzeichnet, dass der gemeinsame Kühlwasserstrom zuerst durch die eine Reaktionszone und sodann, nach optionaler Abkühlung, durch die andere Reaktionszone geführt wird. Dieser Aspekt gestattet einen ressourcenschonenden Einsatz von Kühlwasser und weist gleichzeitig mehr Möglichkeiten zur Einstellung optimaler Temperaturen auf.

Ein achter Aspekt des erfindungsgemäßen Verfahrens ist dadurch gekennzeichnet, dass der gemeinsame Kühlwasserstrom zuerst durch die zweite Reaktionszone und sodann, nach optionaler Abkühlung, durch die erste Reaktionszone geführt wird. Dieser Aspekt gestattet einen ressourcenschonenden Einsatz von Kühlwasser und weist gleichzeitig mehr Möglichkeiten zur Einstellung optimaler Temperaturen auf. Der gemeinsame Kühlwasserstrom bewegt sich dabei im Gegenstrom zu den Edukt- und Produktgasen durch den Synthesereaktor.

Ein neunter Aspekt des erfindungsgemäßen Verfahrens ist dadurch gekennzeichnet, dass der gemeinsame Kühlwasserstrom zuerst durch die erste Reaktionszone und sodann, nach optionaler Abkühlung, durch die zweite Reaktionszone geführt wird. Dieser Aspekt gestattet einen ressourcenschonenden Einsatz von Kühlwasser und weist gleichzeitig mehr Möglichkeiten zur Einstellung optimaler Temperaturen auf. Der gemeinsame Kühlwasserstrom bewegt sich dabei im Gleichstrom zu den Edukt- und Produktgasen durch den Synthesereaktor.

Ein zehnter Aspekt des erfindungsgemäßen Verfahrens ist dadurch gekennzeichnet, dass der erste Kühlwasserstrom und/oder der zweite Kühlwasserstrom und/oder der gemeinsame Kühlwasserstrom im Gleichstrom durch die erste Reaktionszone und/oder die zweite Reaktionszone geführt werden, relativ zu der Gasströmung durch die erste Reaktionszone und/oder die zweite Reaktionszone. Dieser Aspekt betrifft die Strömungsrichtung des Kühlmediums innerhalb einer Reaktionszone.

Ein elfter Aspekt des erfindungsgemäßen Verfahrens ist dadurch gekennzeichnet, dass der erste Kühlwasserstrom und/oder der zweite Kühlwasserstrom und/oder der gemeinsame Kühlwasserstrom im Gegenstrom durch die erste Reaktionszone und/oder die zweite Reaktionszone geführt werden, relativ zu der Gasströmung durch die erste Reaktionszone und/oder die zweite Reaktionszone. Dieser Aspekt betrifft die Strömungsrichtung des Kühlmediums innerhalb einer Reaktionszone.

Ein zwölfter Aspekt des erfindungsgemäßen Verfahrens ist dadurch gekennzeichnet, dass mindestens ein Teil des Abwasserstroms nach optionaler Kühlung als erster Kühlwasserstrom und/oder zweiter Kühlwasserstrom und/oder gemeinsamer Kühlwasserstrom verwendet wird. Dieser Aspekt gestattet einen besonders ressourcenschonenden Einsatz von Kühlwasser.

Ein dreizehnter Aspekt des erfindungsgemäßen Verfahrens ist dadurch gekennzeichnet, dass mindestens ein Teil des ersten Kühlwasserstroms und/oder des zweiten Kühlwasserstroms und/oder des gemeinsamen Kühlwasserstroms beim Passieren der ersten Reaktionszone und/oder der zweiten Reaktionszone mindestens teilweise verdampft und als Dampf oder Dampf-Flüssig-Zweiphasengemisch ausgeleitet wird. Wegen des Phasenübergangs und der damit verbundenen, besonders große Enthalpieänderung wird eine besonders große Kühlwirkung erzielt.

Ein vierzehnter Aspekt des erfindungsgemäßen Verfahrens ist dadurch gekennzeichnet, dass mindestens ein Teil des ersten Einsatzgasstroms und/oder mindestens ein Teil des zu dem DME-Synthesereaktor zurückgeführten Gas-Nebenproduktstroms als erstes fluides Kühlmedium und/oder als zweites fluides Kühlmedium verwendet wird, bevor der mindestens eine Teil des ersten Einsatzgasstroms und/oder der mindestens eine Teil des zu dem DME-Synthesereaktor zurückgeführten Gas-Nebenproduktstroms in den DME-Synthesereaktor eingeleitet wird. Auf diese Weise wird Kühlmedium, beispielsweise Kühlwasser oder Wärmeträgeröl, eingespart und die entsprechenden Gasströme werden vortemperiert.

Ein fünfzehnter Aspekt des erfindungsgemäßen Verfahrens ist dadurch gekennzeichnet, dass mindestens ein Teil des Methanol-Nebenproduktstroms als erstes fluides Kühlmedium in die erste Reaktionszone eingeleitet wird, wobei der Methanol-Nebenproduktstrom erhitzt wird und sodann in den Zwischenraum und/oder in die Katalysatorschüttungen in der zweiten Reaktionszone eingeleitet wird. Auf diese Weise kann speziell die Ausbeute bei der zweiten Teilreaktion, der DME-Synthese, erhöht werden. Gleichzeitig wird Kühlmedium, beispielsweise Kühlwasser oder Wärmeträgeröl, eingespart und der Methanol-Nebenproduktstrom wird vortemperiert.

Ein sechzehnter Aspekt des erfindungsgemäßen Verfahrens ist dadurch gekennzeichnet, dass zunächst die zweite Reaktionszone (DME-Synthese) mit von außen zugeführtem Methanol in Betrieb genommen wird und dass Synthesegas als Kühlmedium durch die zweite Reaktionszone geführt, um die gewünschte Temperatur zu erreichen und sodann die die erste Reaktionszone (Methanolsynthese) in Betrieb zu nehmen. Auf diese Weise kann das erfindungsgemäße Verfahren besonders schnell in Betrieb genommen werden.

Ein siebzehnter Aspekt des erfindungsgemäßen Verfahrens ist dadurch gekennzeichnet, dass eine maximale Reaktionstemperatur von 400 °C in der zweiten Reaktionszone nicht überschritten wird. Hierdurch kann das Ausmaß der Methanisierung gering gehalten werden.

Ein achtzehnter Aspekt des erfindungsgemäßen Verfahrens ist dadurch gekennzeichnet, dass die Temperatur in der ersten Reaktionszone zwischen 180 und 350 °C, meist bevorzugt zwischen 200 und 280 °C liegt und dass die Temperatur in der zweiten Reaktionszone zwischen 220 und 350 °C, weiter bevorzugt zwischen 240 und 320 °C, meist bevorzugt zwischen 260 und 300 °C liegt. Untersuchungen haben ergeben, dass in diesen Temperaturbereichen hohe Ausbeuten an DME und geringe Ausbeuten an Nebenprodukten erhalten werden.

Ein neunzehnter Aspekt des erfindungsgemäßen Verfahrens ist dadurch gekennzeichnet, dass der Betriebsdruck des DME-Synthesereaktors gleich oder größer als 90 bar, absolut und die minimale Betriebstemperatur beider Reaktionszonen gleich oder größer als 250 °C, bevorzugt gleich oder größer als 260 °C beträgt. Auf diese Weise kann der DME-Synthesereaktor betrieben werden, ohne dass eine Kondensation im Reaktorinneren, insbesondere innerhalb der ersten und zweiten Reaktionszone auftritt.

### Ausführungsbeispiele

Weitere Merkmale, Vorteile und Anwendungsmöglichkeiten der Erfindung ergeben sich aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbezügen.

Es zeigen:
- Fig. 1: eine schematische Darstellung des erfindungsgemäßen Verfahrens in einer beispielshaften Ausgestaltung eines DME-Synthesereaktors,
- Fig. 2: eine Detailansicht einer ersten Reaktionszone des erfindungsgemäßen DME-Synthesereaktors,
- Fig. 3: ein konstruktives Detail der Thermoplatten (schematisch).

Im Folgenden ist die Angabe "nicht gezeigt" so zu verstehen, dass ein Element in der besprochenen Abbildung nicht grafisch dargestellt, aber gemäß der Beschreibung trotzdem vorhanden ist.

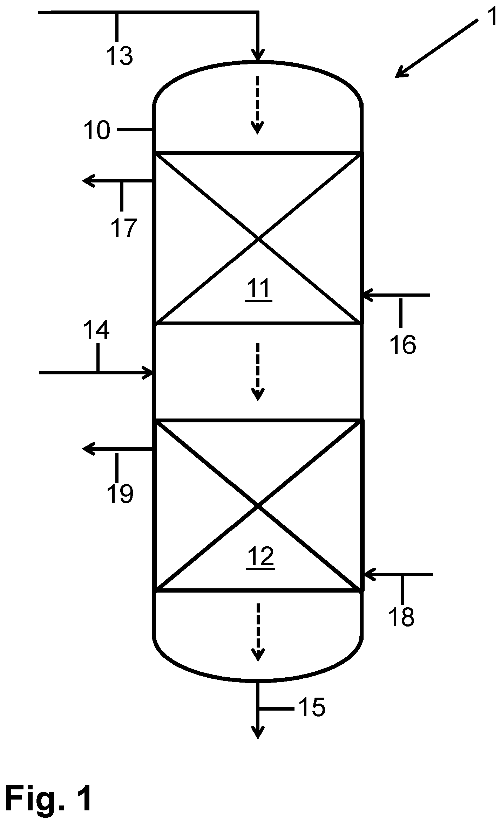

Figur 1 zeigt eine schematische Darstellung des erfindungsgemäßen Verfahrens in einer beispielshaften Ausgestaltung eines DME-Synthesereaktors 1. In einem gemeinsamen, bezüglich seiner Längsachse senkrecht stehenden, drucktragenden Mantelrohr 10 sind eine erste Reaktionszone 11 und eine zweite Reaktionszone 12 übereinander angeordnet. Über eine Leitung 13 wird Synthesegas als Einsatzstrom in den DME-Synthesereaktor 1 eingeleitet und durchströmt diesen von oben nach unten; dies ist durch gestrichelte Strömungspfeile angedeutet. Der Einsatzstrom tritt über einen ersten Einlass (nicht gezeigt) in die erste Reaktionszone 11 ein, deren Katalysatorzonen mit einem für die Methanolsynthese aktiven, kommerziell erhältlichen, partikelförmigen Katalysator gefüllt sind.

In der ersten Reaktionszone 11 wird der Einsatzstrom unter Methanolsynthesebedingungen zumindest teilweise in Methanol umgewandelt. Der mindestens teilumgewandelte, Methanol umfassende Einsatzstrom wird über einen ersten Auslass (nicht gezeigt) als erster, Methanol enthaltender Produktgasstrom aus der ersten Reaktionszone 11 ausgeleitet und sodann über einen zweiten Einlass (nicht gezeigt) für das erste Produktgas in eine zweite Reaktionszone 12 eingeleitet.

In der zweiten Reaktionszone 12 wird der erste, Methanol enthaltende Produktgasstrom unter DME-Synthesebedingungen zumindest teilweise in DME umgewandelt. Dabei wird ein zweiter, DME enthaltender Produktgasstrom gewonnen, der aus der zweiten Reaktionszone über einen zweiten Auslass (nicht gezeigt) und über eine Leitung 15 (Produktauslass) am Mantelrohr 10 aus dem DME-Synthesereaktor 1 ausgeleitet und einer nicht gezeigten nach mindestens einem thermischen Trennverfahren arbeitenden Trennvorrichtung zugeführt.

In der Trennvorrichtung erfolgt ein Auftrennen des DME enthaltenden Produktgasstroms in einen DME-Endproduktstrom, einen Gas-Nebenproduktstrom, der nicht umgesetzte Kohlenoxide und Wasserstoff enthält, einen Methanol-Nebenproduktstrom und einen Abwasserstrom. Der DME-Endproduktstrom wird in einem Beispiel einer DME-Lagerung, DME-Feinreinigung und/oder DME-Weiterverarbeitung zugeführt (alle nicht gezeigt). Der Gas-Nebenproduktstrom wird bevorzugt mindestens teilweise zum DME-Synthesereaktor 1 zurückgeführt und über die Leitung 13 in diesen eingeleitet. Der Methanol-Nebenproduktstrom wird bevorzugt mindestens teilweise zum DME-Synthesereaktor 1 zurückgeführt, über eine Leitung 14 in diesen eingeleitet und direkt der zweiten Reaktionszone zugeführt. Der Abwasserstrom wird in einem Beispiel aus dem Verfahren ausgeleitet. In einem weiteren Beispiel wird der Abwasserstrom nach optionaler Abkühlung als Kühlmedium zum DME-Synthesereaktor 1 zurückgeführt.

Über eine Leitung 16 und einen nicht gezeigten Einlass wird ein erstes fluides Kühlmedium in die erste Reaktionszone 11 eingeleitet. Das erste fluide Kühlmedium absorbiert mindestens einen Teil der Reaktionswärme der exotherm verlaufenden Methanolsynthese und wird dabei selbst erwärmt. Das erwärmte erste fluide Kühlmedium wird über einen nicht gezeigten Auslass und eine Leitung 17 aus der ersten Reaktionszone 11 ausgeleitet.

Über eine Leitung 18 und einen nicht gezeigten Einlass wird ein zweites fluides Kühlmedium in die zweite Reaktionszone 12 eingeleitet. Das zweite fluide Kühlmedium absorbiert mindestens einen Teil der Reaktionswärme der exotherm verlaufenden DME-Synthese aus Methanol und wird dabei selbst erwärmt. Das erwärmte zweite fluide Kühlmedium wird über einen nicht gezeigten Auslass und eine Leitung 19 aus der zweiten Reaktionszone 12 ausgeleitet.

In einem Beispiel beträgt der Druck im erfindungsgemäßen Synthesereaktor 40 bis 90 bar, absolut, bevorzugt 60 bis 80 bar, absolut. In einem Beispiel wird bevorzugt wird ein Synthesegas in den DME-Synthesereaktor 1 eingeleitet, dessen CO-Konzentration höher ist als dessen CO₂-Konzentration, da hiermit weniger Wasser entsteht und die Selektivität zu DME besser ist. Es können aber auch andere Synthesegase verwendet werden, beispielsweise auch Gemische von CO₂ und Wasserstoff mit nur geringer oder keiner Beimischung von CO.

In einem Beispiel beträgt die Temperatur in der ersten Reaktionszone 11 bevorzugt zwischen 180 und 350 °C, meist bevorzugt zwischen 200 und 280 °C. In einem Beispiel beträgt die Temperatur in der zweiten Reaktionszone 12 zwischen 220 und 350 °C, weiter bevorzugt zwischen 240 und 320 °C, meist bevorzugt zwischen 260 und 300 °C. Untersuchungen haben ergeben, dass in diesen Temperaturbereichen hohe Ausbeuten an DME und geringe Ausbeuten an Nebenprodukten erhalten werden.

Als Kühlmittel können spezielle Wärmeträgerfluide, aber auch die Edukt- und/oder Produktströme der Reaktionszonen verwendet werden. In einem Beispiel wird Wasser als Kühlmedium verwendet, wobei beispielsweise Frischwasser oder bei der Reaktion erzeugtes Wasser oder Mischungen beider verwendet werden können. In einem weiteren Beispiel wird kaltes Synthesegas als Kühlmedium verwendet, das dabei selber vorgewärmt wird, so dass sich der Bedarf des Verfahrens an Heizenergie verringert. In einem weiteren Beispiel wird ein Methanol-Nebenproduktstrom aus der Trennvorrichtung als Kühlmedium verwendet. Hierdurch verringert sich der Energiebedarf der nachgeschalteten Herstellung von Reinmethanol.

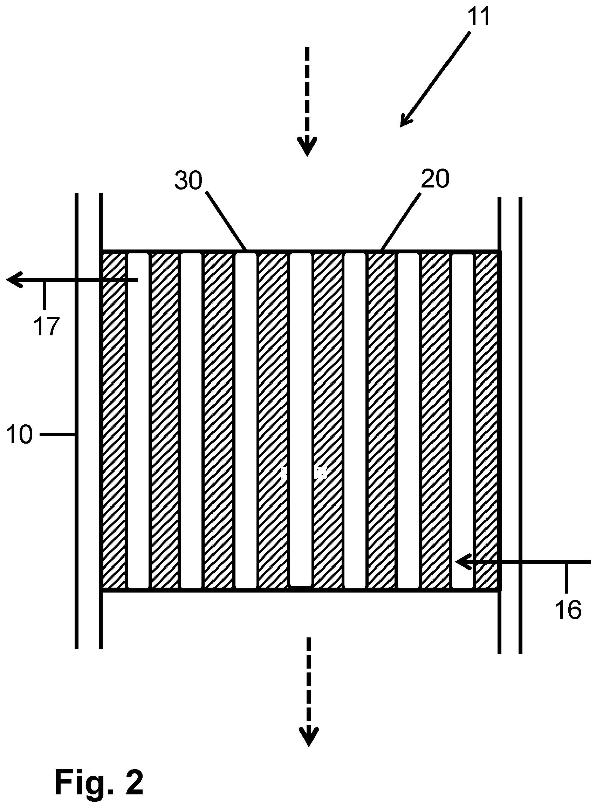

Fig. 2 zeigt eine Detailansicht einer ersten Reaktionszone 11 des erfindungsgemäßen DME-Synthesereaktors 1. Die zweite Reaktionszone 12 weist grundsätzlich denselben Aufbau auf, allerdings sind die Katalysatorzonen mit einem für die DME-Synthese aus Methanol aktiven Katalysator gefüllt.

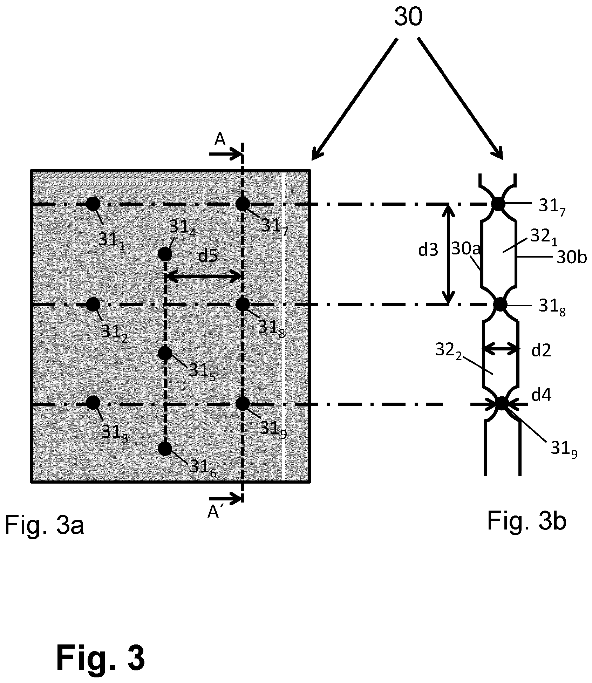

In der ersten Reaktionszone 11 sind Thermoplatten 30 vorzugsweise parallel und gleich beabstandet angeordnet. Der detaillierte Aufbau der Thermoplatten 30 wird unten im Zusammenhang mit Fig. 3 erläutert. Durch die Anordnung der Thermoplatten ergeben sich Zwischenräume 20, die mit Schüttungen eines für die Methanolsynthese aktiven, festen, partikelförmigen Katalysators gefüllt werden. Beide Enden der Zwischenräume sind für Gasströme durchlässig. Das untere Ende der Zwischenräume und bevorzugt auch das obere Ende der Zwischenräume weisen nicht gezeigte Auflagen bzw. Rückhaltevorrichtungen für die Katalysatorschüttungen auf, beispielsweise Siebböden, Lochbleche oder Drahtgewebe. Die bevorzugt an der Eintrittsseite der Gasströmung in die erste Reaktionszone 11 angebrachte Rückhaltevorrichtung kann dabei vorteilhaft auch zur Vergleichmäßigung und Verteilung des Einsatzgasstroms auf die einzelnen Katalysatorbetten dienen.

Über Leitung 16 und einen nicht gezeigten Einlass wird ein erstes fluides Kühlmedium in die Thermoplatten 30 in der ersten Reaktionszone 11 eingeleitet. Das erste fluide Kühlmedium absorbiert mindestens einen Teil der Reaktionswärme der exotherm verlaufenden Methanolsynthese und wird dabei selbst erwärmt. Das erwärmte erste fluide Kühlmedium wird über einen nicht gezeigten Auslass und Leitung 17 aus der ersten Reaktionszone 11 ausgeleitet. Das Einleiten und Ausleiten des ersten fluiden Kühlmediums in die bzw. aus den Thermoplatten 30 erfolgt über ein nicht gezeigtes Verteilersystem. Dies ist durch die als Pfeile dargestellten Leitungen 16 und 17 angedeutet.

Figur 3a zeigt eine x-y-Ansicht auf eine Thermoplatte 30 über die Fläche eines Bleches, welche eine Seite der Thermoplatte 30 bildet. Die Punkte 31₁ bis 31₉ stellen die sogenannten Schweißpunkte dar, mit denen das Blech mit dem nicht dargestellten Blech auf der gegenüberliegenden Seite durch eine zusätzliche Punktschweißung verbunden ist. Die Punkte 31₁ bis 31₃, 31₃ bis 31₆ und 31₆ bis 31₉ liegen jeweils auf einer Geraden und wobei alternierend die Punkte jeder zweiten Gerade wieder auf einer Geraden in der jeweils anderen Dimension liegen. Die Geraden verlaufen zueinander parallel und weisen den Abstand d5 auf.

Indem die Thermoplatte nicht nur an den Rändern von zwei übereinander gelegten Blechen miteinander verschweißt ist, sondern sich zusätzliche Schweißpunkte 31₁ bis 31₉ darauf befinden, ergibt sich in der x-z-Ansicht durch eine Thermoplatte die in Figur 3b gezeigte Schnittdarstellung entlang der Geraden A - A'. Zwischen den einzelnen Punktschweißungen 31₁ bis 31₉ bilden sich Kanäle 32₁ und 32₂ aus, die in der Regel durch in eine Druckumformung, besonders bevorzugt durch eine Innenhochdruckumformung, erzeugt werden. Der Durchmesser eines solchen Kanals 32₁ oder 32₂ d2 beschreibt den Abstand zwischen den beiden Blechen 30a und 30b an der maximalen Kanalausbildung, während der Durchmesser d4 die Dicke des Schweißpunktes 31₁ bis 31₉ bezeichnet. Der Abstand zwischen zwei Schweißpunkten 31₁ bis 31₉, der genau auch dem Abstand von zwei Schweißpunkten 31₁ bis 31₉ auf einer Gerade entspricht, entspricht d3. Vorzugsweise gilt d3 > d2 > d4.

### Zahlenbeispiele

In der nachfolgenden Tabelle werden beispielhafte Betriebsbedingungen für den erfindungsgemäßen DME-Synthesereaktor zusammengestellt, unter denen ein Reaktorbetrieb ohne Taupunktsunterschreitung und mithin ohne Kondensation im Reaktor ermöglicht wird. Dies ist wichtig, weil hierdurch die Standzeit und mögliche Betriebsdauer des Katalysators nicht beeinträchtigt wird.

**Tabelle: Beispielhafte Betriebsbedingungen für den DME-Synthesereaktor**

| | Anteil / mol% | p / bar | Taupunkt / °C | p / bar | Taupunkt / °C |
|---|---|---|---|---|---|
| Gesamtdruck | | 90,0 | | 60,0 | |
| DME | 40,0 | 36,0 | | 24,0 | |
| Wasser | 40,0 | 36,0 | 250 | 24,0 | 225 |
| Methanol | 20,0 | 18,0 | | 12,0 | |

Im Ergebnis ist es vorteilhaft, den Betriebsdruck des DME-Synthesereaktors gemäß der Erfindung gleich oder größer als 90 bar, absolut und die minimale Betriebstemperatur beider Reaktionszonen gleich oder größer als 250 °C, bevorzugt gleich oder größer als 260 °C zu wählen.

### Bezugszeichenliste

- 1: DME-Synthesereaktor
- 10: Mantelrohr
- 11: erste Reaktionszone
- 12: zweite Reaktionszone
- 13: Leitung (Einsatzgasstrom)
- 14: Leitung (Methanol-Seitenzufuhr)
- 15: Leitung (Produktgasstrom)
- 16: Leitung (Einlass erstes fluides Kühlmedium)
- 17: Leitung (Auslass erstes fluides Kühlmedium)
- 18: Leitung (Einlass zweites fluides Kühlmedium)
- 19: Leitung (Auslass zweites fluides Kühlmedium)
- 20: Katalysatorschüttungen in den Zwischenräumen zwischen Thermoplatten (schraffiert)
- 30: Thermoplatten
- 31: Punktschweißungen
- 32: freier Innenraum

## Patentansprüche

1. Verfahren zum Herstellen von Dimethylether (DME) aus einem Kohlenoxide und Wasserstoff enthaltenden Synthesegas, umfassend folgende Schritte:
(a) Bereitstellen eines DME-Synthesereaktors, umfassend folgende Bestandteile:
(a1) eine erste Reaktionszone mit einem ersten Einlass für Synthesegas als erstes Einsatzgas und einem ersten Auslass für ein erstes, Methanol enthaltendes Produktgas, wobei die erste Reaktionszone eine Vielzahl von Thermoplatten umfasst, die so angeordnet sind, dass:
- jeweils zwischen zwei benachbarten Thermoplatten eine Schüttung eines festen, partikelförmigen, für die Methanolsynthese aus dem Synthesegas aktiven Katalysators angeordnet ist, die von dem Synthesegas durchströmbar ist,
- die Thermoplatten einen Einlass für ein erstes fluides Kühlmedium und einen Auslass für das erste fluide Kühlmedium aufweisen und in ihrem Inneren für das erste fluide Kühlmedium durchströmbar sind;
(a2) eine zweite Reaktionszone mit einem zweiten Einlass für das erste Produktgas und einem zweiten Auslass für ein zweites, DME enthaltendes Produktgas, wobei die zweite Reaktionszone eine Vielzahl von Thermoplatten umfasst, die so angeordnet sind, dass:
- jeweils zwischen zwei benachbarten Thermoplatten eine Schüttung eines festen, partikelförmigen, für die DME-Synthese aus Methanol aktiven Katalysators angeordnet ist, die von dem ersten Produktgas durchströmbar ist,
- die Thermoplatten einen Einlass für ein zweites fluides Kühlmedium und einen Auslass für das zweite fluide Kühlmedium aufweisen und in ihrem Inneren für das zweite fluide Kühlmedium durchströmbar sind;
(a3) ein äußeres, drucktragendes Mantelrohr, in dessen Inneren die erste Reaktionszone und durch einen Zwischenraum dazu beabstandet die zweite Reaktionszone angeordnet sind, wobei das Mantelrohr an dem der ersten Reaktionszone benachbarten Ende einen Edukteinlass zum Einleiten von Synthesegas als erstes Einsatzgas und an dem der zweiten Reaktionszone benachbarten Ende einen Produktauslass zum Ausleiten des zweiten, DME enthaltenden Produktgases aufweist;
(b) Einleiten eines Synthesegasstroms als erster Einsatzgasstrom in den DME-Synthesereaktor über den Edukteinlass am Mantelrohr und über den ersten Einlass in die erste Reaktionszone;
(c) Umsetzen des ersten Einsatzgasstroms in der ersten Reaktionszone unter Methanolsynthesebedingungen;
(d) Ausleiten eines ersten, Methanol enthaltenden Produktgasstroms aus der ersten Reaktionszone;
(e) Einleiten des ersten, Methanol enthaltendes Produktgasstroms in die zweite Reaktionszone;
(f) Umsetzen des ersten Produktgasstroms in der zweiten Reaktionszone unter DME-Synthesebedingungen;
(g) Ausleiten eines zweiten, DME enthaltenden Produktgasstroms aus der zweiten Reaktionszone über den zweiten Auslass und über den Produktauslass am Mantelrohr aus dem DME-Synthesereaktor;
(h) Zuführen des DME enthaltenden Produktgasstroms zu einer nach mindestens einem thermischen Trennverfahren arbeitenden Trennvorrichtung, Auftrennen des DME enthaltenden Produktgasstroms in der Trennvorrichtung in einen DME-Endproduktstrom, einen Gas-Nebenproduktstrom, der nicht umgesetzte Kohlenoxide und Wasserstoff enthält, einen Methanol-Nebenproduktstrom und einen Abwasserstrom.

2. Verfahren nach Anspruch 1, **dadurch gekennzeichnet, dass** ein erster Teil des Gas-Nebenproduktstroms zu dem DME-Synthesereaktor zurückgeführt und gemeinsam mit dem ersten Einsatzgasstrom in den DME-Synthesereaktor eingeleitet wird.

3. Verfahren nach Anspruch 1 oder 2, **dadurch gekennzeichnet, dass** ein zweiter Teil des Gas-Nebenproduktstroms als Spülstrom (Purge) aus dem Verfahren ausgeleitet wird.

4. Verfahren nach einem der vorigen Ansprüche, **dadurch gekennzeichnet, dass** mindestens ein Teil des Methanol-Nebenproduktstroms zu dem DME-Synthesereaktor zurückgeführt und in den Zwischenraum und/oder in die Katalysatorschüttungen in der zweiten Reaktionszone eingeleitet wird.

5. Verfahren nach einem der vorigen Ansprüche, **dadurch gekennzeichnet, dass** als erstes fluides Kühlmedium ein erster Kühlwasserstrom und als zweites fluides Kühlmedium ein zweiter Kühlwasserstrom verwendet wird.

6. Verfahren nach einem der vorigen Ansprüche, **dadurch gekennzeichnet, dass** als erstes fluides Kühlmedium und als zweites fluides Kühlmedium ein gemeinsamer Kühlwasserstrom verwendet wird.

7. Verfahren nach Anspruch 6, **dadurch gekennzeichnet, dass** der gemeinsame Kühlwasserstrom zuerst durch die eine Reaktionszone und sodann, nach optionaler Abkühlung, durch die andere Reaktionszone geführt wird.

8. Verfahren nach Anspruch 7, **dadurch gekennzeichnet, dass** der gemeinsame Kühlwasserstrom zuerst durch die zweite Reaktionszone und sodann, nach optionaler Abkühlung, durch die erste Reaktionszone geführt wird.

9. Verfahren nach Anspruch 7, **dadurch gekennzeichnet, dass** der gemeinsame Kühlwasserstrom zuerst durch die erste Reaktionszone und sodann, nach optionaler Abkühlung, durch die zweite Reaktionszone geführt wird.

10. Verfahren nach einem der Ansprüche 5 bis 9, **dadurch gekennzeichnet, dass** der erste Kühlwasserstrom und/oder der zweite Kühlwasserstrom und/oder der gemeinsame Kühlwasserstrom im Gleichstrom durch die erste Reaktionszone und/oder die zweite Reaktionszone geführt werden, relativ zu der Gasströmung durch die erste Reaktionszone und/oder die zweite Reaktionszone.

11. Verfahren nach einem der Ansprüche 5 bis 9, **dadurch gekennzeichnet, dass** der erste Kühlwasserstrom und/oder der zweite Kühlwasserstrom und/oder der gemeinsame Kühlwasserstrom im Gegenstrom durch die erste Reaktionszone und/oder die zweite Reaktionszone geführt werden, relativ zu der Gasströmung durch die erste Reaktionszone und/oder die zweite Reaktionszone.

12. Verfahren nach einem der vorigen Ansprüche, **dadurch gekennzeichnet, dass** mindestens ein Teil des Abwasserstroms nach optionaler Kühlung als erster Kühlwasserstrom und/oder zweiter Kühlwasserstrom und/oder gemeinsamer Kühlwasserstrom verwendet wird.

13. Verfahren nach einem der vorigen Ansprüche, **dadurch gekennzeichnet, dass** mindestens ein Teil des ersten Kühlwasserstroms und/oder des zweiten Kühlwasserstroms und/oder des gemeinsamen Kühlwasserstroms beim Passieren der ersten Reaktionszone und/oder der zweiten Reaktionszone mindestens teilweise verdampft und als Dampf oder Dampf-Flüssig-Zweiphasengemisch ausgeleitet wird.

14. Verfahren nach einem der vorigen Ansprüche, **dadurch gekennzeichnet, dass** mindestens ein Teil des ersten Einsatzgasstroms und/oder mindestens ein Teil des zu dem DME-Synthesereaktor zurückgeführten Gas-Nebenproduktstroms als erstes fluides Kühlmedium und/oder als zweites fluides Kühlmedium verwendet wird, bevor der mindestens eine Teil des ersten Einsatzgasstroms und/oder der mindestens eine Teil des zu dem DME-Synthesereaktor zurückgeführten Gas-Nebenproduktstroms in den DME-Synthesereaktor eingeleitet wird.

15. Verfahren nach einem der vorigen Ansprüche, **dadurch gekennzeichnet, dass** mindestens ein Teil des Methanol-Nebenproduktstroms als erstes fluides Kühlmedium in die erste Reaktionszone eingeleitet wird, wobei der Methanol-Nebenproduktstrom erhitzt wird und sodann in den Zwischenraum und/oder in die Katalysatorschüttungen in der zweiten Reaktionszone eingeleitet wird.

16. Verfahren nach einem der vorigen Ansprüche, **dadurch gekennzeichnet, dass** zunächst die zweite Reaktionszone (DME-Synthese) mit von außen zugeführtem Methanol in Betrieb genommen wird und dass Synthesegas als Kühlmedium durch die zweite Reaktionszone geführt, um die gewünschte Temperatur zu erreichen und sodann die die erste Reaktionszone (Methanolsynthese) in Betrieb zu nehmen.

17. Verfahren nach einem der vorigen Ansprüche, **dadurch gekennzeichnet, dass** eine maximale Reaktionstemperatur von 400 °C in der zweiten Reaktionszone nicht überschritten wird.

18. Verfahren nach einem der vorigen Ansprüche, **dadurch gekennzeichnet, dass** die Temperatur in der ersten Reaktionszone zwischen 180 und 350 °C, meist bevorzugt zwischen 200 und 280 °C liegt und dass die Temperatur in der zweiten Reaktionszone zwischen 220 und 350 °C, weiter bevorzugt zwischen 240 und 320 °C, meist bevorzugt zwischen 260 und 300 °C liegt.

19. Verfahren nach einem der vorigen Ansprüche, **dadurch gekennzeichnet, dass** der Betriebsdruck des DME-Synthesereaktors gleich oder größer als 90 bar, absolut und die minimale Betriebstemperatur beider Reaktionszonen gleich oder größer als 250 °C, bevorzugt gleich oder größer als 260 °C beträgt.

## Claims

1. A method for producing dimethyl ether (DME) from a synthesis gas containing carbon oxides and hydrogen, comprising the following steps:
(a) providing a DME synthesis reactor, comprising the following components:
(a1) a first reaction zone with a first inlet for synthesis gas as a first feed gas and a first outlet for a first, methanol-containing product gas, wherein the first reaction zone comprises a plurality of thermo-plates, which are arranged such that:
- between every two adjacent thermo-plates, a bed of a solid, particulate catalyst active for methanol synthesis from the synthesis gas is arranged, through which the synthesis gas can flow,
- the thermo-plates have an inlet for a first fluid cooling medium and an outlet for the first fluid cooling medium and are traversable in their interior by the first fluid cooling medium;
(a2) a second reaction zone with a second inlet for the first product gas and a second outlet for a second, DME-containing product gas, wherein the second reaction zone comprises a plurality of thermo-plates, which are arranged such that:
- between every two adjacent thermo-plates, a bed of a solid, particulate catalyst active for DME synthesis from methanol is arranged, through which the first product gas can flow,
- the thermo-plates have an inlet for a second fluid cooling medium and an outlet for the second fluid cooling medium and are traversable in their interior by the second fluid cooling medium;
(a3) an outer, pressure-bearing shell tube, in the interior of which the first reaction zone and, spaced therefrom by an intermediate space, the second reaction zone are arranged, wherein the shell tube has at the end adjacent to the first reaction zone a reactant inlet for introducing synthesis gas as a first feed gas and at the end adjacent to the second reaction zone a product outlet for discharging the second, DME-containing product gas;
(b) introducing a synthesis gas stream as a first feed gas stream into the DME synthesis reactor via the reactant inlet on the shell tube and via the first inlet into the first reaction zone;
(c) reacting the first feed gas stream in the first reaction zone under methanol synthesis conditions;
(d) discharging a first, methanol-containing product gas stream from the first reaction zone;
(e) introducing the first, methanol-containing product gas stream into the second reaction zone;
(f) reacting the first product gas stream in the second reaction zone under DME synthesis conditions;
(g) discharging a second, DME-containing product gas stream from the second reaction zone via the second outlet and via the product outlet on the shell tube from the DME synthesis reactor;
(h) feeding the DME-containing product gas stream to a separation device operating according to at least one thermal separation method, separating the DME-containing product gas stream in the separation device into a DME final product stream, a gas by-product stream containing unreacted carbon oxides and hydrogen, a methanol by-product stream, and a wastewater stream.

2. The method according to claim 1, **characterized in that** a first part of the gas by-product stream is recycled to the DME synthesis reactor and is introduced together with the first feed gas stream into the DME synthesis reactor.

3. The method according to claim 1 or 2, **characterized in that** a second part of the gas by-product stream is discharged from the process as a purge stream.

4. The method according to one of the preceding claims, **characterized in that** at least a part of the methanol by-product stream is recycled to the DME synthesis reactor and is introduced into the intermediate space and/or into the catalyst beds in the second reaction zone.

5. The method according to one of the preceding claims, **characterized in that** a first cooling water stream is used as a first fluid cooling medium and a second cooling water stream is used as a second fluid cooling medium.

6. The method according to one of the preceding claims, **characterized in that** a common cooling water stream is used as the first fluid cooling medium and as the second fluid cooling medium.

7. The method according to claim 6, **characterized in that** the common cooling water stream is first passed through the one reaction zone and then, after optional cooling, through the other reaction zone.

8. The method according to claim 7, **characterized in that** the common cooling water stream is first passed through the second reaction zone and then, after optional cooling, through the first reaction zone.

9. The method according to claim 7, **characterized in that** the common cooling water stream is first passed through the first reaction zone and then, after optional cooling, through the second reaction zone.

10. The method according to one of claims 5 to 9, **characterized in that** the first cooling water stream and/or the second cooling water stream and/or the common cooling water stream are passed in cocurrent flow through the first reaction zone and/or the second reaction zone, relative to the gas flow through the first reaction zone and/or the second reaction zone.

11. The method according to one of claims 5 to 9, **characterized in that** the first cooling water stream and/or the second cooling water stream and/or the common cooling water stream are passed in countercurrent flow through the first reaction zone and/or the second reaction zone, relative to the gas flow through the first reaction zone and/or the second reaction zone.

12. The method according to one of the preceding claims, **characterized in that** at least a part of the wastewater stream is used, after optional cooling, as a first cooling water stream and/or a second cooling water stream and/or a common cooling water stream.

13. The method according to one of the preceding claims, **characterized in that** at least a part of the first cooling water stream and/or the second cooling water stream and/or the common cooling water stream is at least partially vaporized when passing through the first reaction zone and/or the second reaction zone and is discharged as steam or a steam-liquid two-phase mixture.

14. The method according to one of the preceding claims, **characterized in that** at least a part of the first feed gas stream and/or at least a part of the gas by-product stream recycled to the DME synthesis reactor is used as a first fluid cooling medium and/or as a second fluid cooling medium, before the at least one part of the first feed gas stream and/or the at least one part of the gas by-product stream recycled to the DME synthesis reactor is introduced into the DME synthesis reactor.

15. The method according to one of the preceding claims, **characterized in that** at least a part of the methanol by-product stream is introduced as a first fluid cooling medium into the first reaction zone, wherein the methanol by-product stream is heated and is then introduced into the intermediate space and/or into the catalyst beds in the second reaction zone.

16. The method according to one of the preceding claims, **characterized in that** first the second reaction zone (DME synthesis) is put into operation with externally supplied methanol and that synthesis gas is passed as a cooling medium through the second reaction zone to achieve the desired temperature and then the first reaction zone (methanol synthesis) is put into operation.

17. The method according to one of the preceding claims, **characterized in that** a maximum reaction temperature of 400 °C is not exceeded in the second reaction zone.

18. The method according to one of the preceding claims, **characterized in that** the temperature in the first reaction zone is between 180 and 350 °C, mostly preferably between 200 and 280 °C, and that the temperature in the second reaction zone is between 220 and 350 °C, more preferably between 240 and 320 °C, most preferably between 260 and 300 °C.

19. The method according to one of the preceding claims, **characterized in that** the operating pressure of the DME synthesis reactor is equal to or greater than 90 bar, absolute, and the minimum operating temperature of both reaction zones is equal to or greater than 250 °C, preferably equal to or greater than 260 °C.

## Revendications

1. Procédé de production de diméthyléther (DME) à partir d'un gaz de synthèse contenant des oxydes de carbone et de l'hydrogène, comprenant les étapes suivantes :
(a) la fourniture d'un réacteur de synthèse de DME, comprenant les composants suivants :
(a1) une première zone de réaction avec une première entrée pour le gaz de synthèse en tant que premier gaz d'alimentation et une première sortie pour un premier gaz de produit contenant du méthanol, la première zone de réaction comprenant une pluralité de plaques thermiques, qui sont agencées de telle sorte que :
- entre chaque deux plaques thermiques adjacentes, est agencé un lit d'un catalyseur solide, particulaire, actif pour la synthèse du méthanol à partir du gaz de synthèse, qui peut être traversé par le gaz de synthèse,
- les plaques thermiques présentent une entrée pour un premier fluide de refroidissement et une sortie pour le premier fluide de refroidissement et peuvent être traversées dans leur intérieur par le premier fluide de refroidissement ;
(a2) une deuxième zone de réaction avec une deuxième entrée pour le premier gaz de produit et une deuxième sortie pour un deuxième gaz de produit contenant du DME, la deuxième zone de réaction comprenant une pluralité de plaques thermiques, qui sont agencées de telle sorte que :
- entre chaque deux plaques thermiques adjacentes, est agencé un lit d'un catalyseur solide, particulaire, actif pour la synthèse de DME à partir du méthanol, qui peut être traversé par le premier gaz de produit,
- les plaques thermiques présentent une entrée pour un deuxième fluide de refroidissement et une sortie pour le deuxième fluide de refroidissement et peuvent être traversées dans leur intérieur par le deuxième fluide de refroidissement ;
(a3) une calandre extérieure, résistante à la pression, à l'intérieur de laquelle sont agencées la première zone de réaction et, espacée de celle-ci par un espace intermédiaire, la deuxième zone de réaction, la calandre présentant, à l'extrémité adjacente à la première zone de réaction, une entrée de réactifs pour l'introduction de gaz de synthèse en tant que premier gaz d'alimentation et, à l'extrémité adjacente à la deuxième zone de réaction, une sortie de produit pour l'évacuation du deuxième gaz de produit contenant du DME ;
(b) l'introduction d'un courant de gaz de synthèse en tant que premier courant de gaz d'alimentation dans le réacteur de synthèse de DME via l'entrée de réactifs sur la calandre et via la première entrée dans la première zone de réaction ;
(c) la conversion du premier courant de gaz d'alimentation dans la première zone de réaction dans des conditions de synthèse de méthanol ;
(d) l'évacuation d'un premier courant de gaz de produit contenant du méthanol de la première zone de réaction ;
(e) l'introduction du premier courant de gaz de produit contenant du méthanol dans la deuxième zone de réaction ;
(f) la conversion du premier courant de gaz de produit dans la deuxième zone de réaction dans des conditions de synthèse de DME ;
(g) l'évacuation d'un deuxième courant de gaz de produit contenant du DME de la deuxième zone de réaction via la deuxième sortie et via la sortie de produit sur la calandre hors du réacteur de synthèse de DME ;
(h) l'amenée du courant de gaz de produit contenant du DME à un dispositif de séparation fonctionnant selon au moins un procédé de séparation thermique, la séparation du courant de gaz de produit contenant du DME dans le dispositif de séparation en un courant de produit final de DME, un courant de sous-produit gazeux contenant des oxydes de carbone et de l'hydrogène n'ayant pas réagi, un courant de sous-produit de méthanol et un courant d'eaux usées.

2. Procédé selon la revendication 1, **caractérisé en ce qu'**une première partie du courant de sous-produit gazeux est recyclée vers le réacteur de synthèse de DME et est introduite conjointement avec le premier courant de gaz d'alimentation dans le réacteur de synthèse de DME.

3. Procédé selon la revendication 1 ou 2, **caractérisé en ce qu'**une deuxième partie du courant de sous-produit gazeux est évacuée du procédé en tant que courant de purge.

4. Procédé selon l'une des revendications précédentes, **caractérisé en ce qu'**au moins une partie du courant de sous-produit de méthanol est recyclée vers le réacteur de synthèse de DME et est introduite dans l'espace intermédiaire et/ou dans les lits de catalyseur dans la deuxième zone de réaction.

5. Procédé selon l'une des revendications précédentes, **caractérisé en ce qu'**un premier courant d'eau de refroidissement est utilisé comme premier fluide de refroidissement et un deuxième courant d'eau de refroidissement est utilisé comme deuxième fluide de refroidissement.

6. Procédé selon l'une des revendications précédentes, **caractérisé en ce qu'**un courant d'eau de refroidissement commun est utilisé comme premier fluide de refroidissement et comme deuxième fluide de refroidissement.

7. Procédé selon la revendication 6, **caractérisé en ce que** le courant d'eau de refroidissement commun est d'abord acheminé à travers l'une zone de réaction, puis, après refroidissement optionnel, à travers l'autre zone de réaction.

8. Procédé selon la revendication 7, **caractérisé en ce que** le courant d'eau de refroidissement commun est d'abord acheminé à travers la deuxième zone de réaction, puis, après refroidissement optionnel, à travers la première zone de réaction.

9. Procédé selon la revendication 7, **caractérisé en ce que** le courant d'eau de refroidissement commun est d'abord acheminé à travers la première zone de réaction, puis, après refroidissement optionnel, à travers la deuxième zone de réaction.

10. Procédé selon l'une des revendications 5 à 9, **caractérisé en ce que** le premier courant d'eau de refroidissement et/ou le deuxième courant d'eau de refroidissement et/ou le courant d'eau de refroidissement commun sont acheminés à co-courant à travers la première zone de réaction et/ou la deuxième zone de réaction, par rapport à l'écoulement de gaz à travers la première zone de réaction et/ou la deuxième zone de réaction.

11. Procédé selon l'une des revendications 5 à 9, **caractérisé en ce que** le premier courant d'eau de refroidissement et/ou le deuxième courant d'eau de refroidissement et/ou le courant d'eau de refroidissement commun sont acheminés à contre-courant à travers la première zone de réaction et/ou la deuxième zone de réaction, par rapport à l'écoulement de gaz à travers la première zone de réaction et/ou la deuxième zone de réaction.

12. Procédé selon l'une des revendications précédentes, **caractérisé en ce qu'**au moins une partie du courant d'eaux usées est utilisée, après refroidissement optionnel, comme premier courant d'eau de refroidissement et/ou deuxième courant d'eau de refroidissement et/ou courant d'eau de refroidissement commun.

13. Procédé selon l'une des revendications précédentes, **caractérisé en ce qu'**au moins une partie du premier courant d'eau de refroidissement et/ou du deuxième courant d'eau de refroidissement et/ou du courant d'eau de refroidissement commun est au moins partiellement vaporisée lors du passage de la première zone de réaction et/ou de la deuxième zone de réaction et est évacuée sous forme de vapeur ou d'un mélange diphasique vapeur-liquide.

14. Procédé selon l'une des revendications précédentes, **caractérisé en ce qu'**au moins une partie du premier courant de gaz d'alimentation et/ou au moins une partie du courant de sous-produit gazeux recyclé vers le réacteur de synthèse de DME est utilisée comme premier fluide de refroidissement et/ou comme deuxième fluide de refroidissement, avant que l'au moins une partie du premier courant de gaz d'alimentation et/ou l'au moins une partie du courant de sous-produit gazeux recyclé vers le réacteur de synthèse de DME soit introduite dans le réacteur de synthèse de DME.

15. Procédé selon l'une des revendications précédentes, **caractérisé en ce qu'**au moins une partie du courant de sous-produit de méthanol est introduite en tant que premier fluide de refroidissement dans la première zone de réaction, le courant de sous-produit de méthanol étant chauffé puis introduit dans l'espace intermédiaire et/ou dans les lits de catalyseur dans la deuxième zone de réaction.

16. Procédé selon l'une des revendications précédentes, **caractérisé en ce que** d'abord la deuxième zone de réaction (synthèse de DME) est mise en service avec du méthanol fourni de l'extérieur et **en ce que** du gaz de synthèse est acheminé comme fluide de refroidissement à travers la deuxième zone de réaction, pour atteindre la température souhaitée, puis la première zone de réaction (synthèse de méthanol) est mise en service.

17. Procédé selon l'une des revendications précédentes, **caractérisé en ce qu'**une température de réaction maximale de 400 °C n'est pas dépassée dans la deuxième zone de réaction.

18. Procédé selon l'une des revendications précédentes, **caractérisé en ce que** la température dans la première zone de réaction se situe entre 180 et 350 °C, le plus souvent de préférence entre 200 et 280 °C, et **en ce que** la température dans la deuxième zone de réaction se situe entre 220 et 350 °C, plus préférablement entre 240 et 320 °C, le plus souvent de préférence entre 260 et 300 °C.

19. Procédé selon l'une des revendications précédentes, **caractérisé en ce que** la pression de service du réacteur de synthèse de DME est égale ou supérieure à 90 bar, absolu, et la température de service minimale des deux zones de réaction est égale ou supérieure à 250 °C, de préférence égale ou supérieure à 260 °C.
